# EUROPÄISCHE PATENTANMELDUNG

(11) **EP 4 279 473 A1**
(43) Veröffentlichungstag der Anmeldung: **22.11.2023**
(21) Anmeldenummer: 22173492.4
(22) Anmeldetag: 16.05.2022
(51) Int. Cl.: C04B 41/91, A61C 8/00, A61K 6/818

(54) **ÄTZVERFAHREN**

(71) Anmelder: Morris Hill Ltd., London W1W 5DX (GB)
(72) Erfinder: PLANK, Johann, 80805 München (DE); SCHIEFER, Christopher, 84513 Töging a. Inn (DE); HEIGIS, Frank Gerald, 73630 Remshalden (DE)
(74) Vertreter: Rentsch Partner AG

(57) **Zusammenfassung**

Es wird ein Ätzverfahren zur Oberflächenbehandlung einer Zahnersatzkomponente umfassend ein Implantat, ein keramisches Abutment sowie eine keramische prothetische Versorgung vorgeschlagen. Eine Innenfläche der prothetischen Versorgung und/oder jener Teil des Abutments, welcher zur Aufnahme der prothetischen Versorgung vorgesehen ist, wird mit einem Ätzmittel in Kontakt gebracht wird, wobei das Ätzmittel folgende Edukte enthält: eine mehrprotonige Säure, mindestens ein Alkalisalz und Wasser.

## Beschreibung

Die vorliegende Erfindung betrifft ein Ätzverfahren zur Oberflächenbehandlung einer Zahnersatzkomponente.

### STAND DER TECHNIK

Aus dem Stand der Technik bekannte Säure-Ätzverfahren, im Bereich keramischer Zahnversorgungen, zielen in erster Linie auf die Verbesserung der Osseointegration von Dentalen Implantaten ab. Stellvertretend für eine Vielzahl von Patentpublikationen sei die WO 2020/244814 genannt. Dieses Dokument offenbart ein Zahnimplantat, das verschiedene Bereiche mit unterschiedlichen Oberflächenrauheiten aufweist, wodurch eine verbesserte Anlagerung unterschiedlicher Gewebezellen ermöglicht wird. Das Abutment selbst verfügt über Lamellen. Bei diesen Lamellen handelt es sich vorzugsweise um eine oder mehrere horizontale Mikrorillen in der Oberfläche des Abutments. Diese besondere Ausgestaltung der Oberfläche soll die definitive Befestigung der prothetischen Versorgung verbessern.

Die Anwendung von Säure-Ätzverfahren ist in Zahntechnik Laboren und in Zahnärztlichen Praxen ebenfalls verbreitet. Als Beispiel sei an dieser Stelle die Oberflächenbehandlung von auf Silikat Keramik basierender Kronen genannt, welche in der Regel adhäsiv mit Schmelz und Dentin verbunden werden. Es erfolgt der Einsatz von licht- und dualhärtenden Befestigungskompositen. Voraussetzung hierfür ist, dass die Silikat Keramik mit Flusssäure (HF 5 %) angeätzt wird (ca. 60 Sek.); wodurch eine mikroretentive Oberfläche resultiert, welche im Anschluss mit Silan benetzt wird. Die Flusssäureätzung von glasbasierten Keramiken mit anschließender Silanisierung ist ein seit Jahrzehnten etabliertes und bewährt es Verfahren. Die Flusssäureätzung ist jedoch aus Arbeitsschutzgründen als einer der kritischsten Arbeitsabläufe in der Zahnarztpraxis anzusehen. In der Vergangenheit gab es daher immer wieder Bestrebungen die Flusssäure zu ersetzen. Als Beispiel sei hierfür Ammoniumpolyfluorid genannt. Dieses reagiert mit der Silikat Keramikoberfläche und erzeugt ein raues Ätzmuster.

Von den Silikat Keramiken abzugrenzen sind die Oxidkeramiken auf Basis von Zirkonoxid. Aktuell gängige Praxis in der Keramikvorbehandlung sind Strahlverfahren mit Aluminiumoxid, wobei das Aluminiumoxidpulver in der Regel eine Korngrösse von < 50 Mikrometer aufweist und das Verfahren mit einem Strahldruck zwischen 0.5-1 bar ausgeführt wird. Es ist jedoch bekannt, dass mechanische Oberflächenbehandlungsverfahren, wie das Sandstrahlen einen negativen Einfluss auf die mechanische Stabilität der Oxidkeramik bewirken.

Insgesamt dient die Oberflächenbehandlung der Keramik dazu, den Haftverbund, beispielsweise zwischen Abutment oder Zahnstumpf und Zahnkrone, zu verbessern. Nach aktuellem Wissensstand reichen bereits rund 10 N aus, um zum Beispiel die Krone vom Abutment abzuziehen.

### DARSTELLUNG DER ERFINDUNG

Eine Aufgabe der Erfindung besteht darin, mindestens einen aus dem Stand der Technik bekannten Nachteil zu vermeiden.

Diese Aufgabe wird durch die in den unabhängigen Patentansprüchen definierte Erfindung gelöst.

Das erfindungsgemässe Ätzverfahren betrifft eine Oberflächenbehandlung einer Zahnersatzkomponente, welche einen in den Knochen einbringbaren Teil als Ersatz für die Zahnwurzel umfasst (Implantat), ein Abutment (Stützpfeiler), welches das Verbindungsteil (Mesostruktur) zwischen dem Zahnimplantat und einer prothetischen Versorgung (z.B. Zahnkrone, Brücke) darstellt. Abutment und prothetische Versorgung sind aus einer Keramik. Das erfindungsgemässe Ätzverfahren findet beispielsweise Anwendung bei der Behandlung von Oberflächen, zum Beispiel jenes Bereichs einer Zahnkrone, die auf das Abutment oder einen Zahnstumpf aufgeklebt wird. Es handelt sich das bei um die Innenfläche der Zahnkrone.

Unter prothetischer Versorgung ist jener Teil der Zahnersatzkomponente zu verstehen, welcher oberhalb des Zahnfleisch angeordnet ist. Bei der prothetischen Versorgung handelt es sich beispielsweise um eine Zahnkrone. Die prothetische Versorgung weist eine, einem natürlichen Zahn nachempfundene Oberfläche auf und eine vorerst unbehandelte Innenfläche. Als Innenfläche ist jene Seite der prothetischen Versorgung zu verstehen, welche zur Verbindung mit dem Abutment oder dem Zahnstumpf vorgesehen ist. Unbehandelt bedeutet, dass erst beim Setzen der prothetischen Versorgung die Innenfläche behandelt wird um einen verbesserten Haftverbund zum Beispiel zwischen der Zahnkrone und dem Abutment oder dem Zahnstumpf zu erzielen.

Erfindungsgemäss wird jener Teil des Abutments, die sogenannte Haftfläche, welche zur Aufnahme der prothetischen Versorgung vorgesehen ist und/oder eine Innenfläche der prothetischen Versorgung, beispielsweise die einer Zahnkrone, mit einem Ätzmittel in Kontakt gebracht.

Mit dem erfindungsgemässen Verfahren wird die Oberfläche des Abutments behandelt und/oder eine Innenfläche einer prothetischen Versorgung, sodass eine verbesserte Verbindungzwischen diese beiden Teilen oder zwischen einer prothetischen Versorgung und dem Zahnstumpf resultiert. Die vorbehandelte Oberfläche schafft einerseits einen ausreichenden Haftverbund zwischen dem Abutment und der prothetischen Versorgung oder dem Zahnstumpf und der prothetischen Versorgung, welcher dem Kaudruck mit Biegewechselbelastungen standhält.

Bei der Verbindung des Abutments bzw. Zahnstumpfs mit der prothetischen Versorgung resultiert in erster Linie eine retentive Verbindung, wobei als Haftmittel ein konventioneller Zement eingesetzt wird (zum Beispiel Zinkoxidphosphat oder Glasionomer). Der Verbund zwischen Abutment bzw. Zahnstumpf und prothetischer Versorgung basiert primär auf Friktion. Als Haftflächen stehen die Oberfläche des Abutments bzw. Zahnstumpfs sowie jene Fläche der prothetischen Versorgung, welche zur Verbindung mit dem Abutment bzw. Zahnstumpf vorgesehen ist, zur Verfügung. Angestrebt wird eine definitive Verbindung der prothetischen Versorgung mit dem Abutment bzw. Zahnstumpf. Die Zementierung benötigt eine retentive Oberflächenbehandlung, die mit dem erfindungsgemässen Verfahren geschaffen wird. Eine entsprechende Rauheit der Haftfläche ist für den Verbund und die Haftung entscheidend, denn die prothetische Versorgung wird quasi mit dem Abutment bzw. Zahnstumpf verkeilt.

Anders als das Abutment oder die Innenfläche der prothetischen Versorgung wird der Zahnstumpf, insbesondere dessen Oberfläche nicht mit dem Ätzverfahren behandelt, sondern durch Behandlung mit einem Diamantschleifkörper aufweisend eine mittlere Korngrösse zwischen 15 und 151 Mikrometern (µm). Auf diese Weise wird die gewünschte Rauigkeit erzielt.

Das erfindungsgemässe Verfahren kann zur Oberflächenbehandlung alle Teile einer Zahnersatzkomponente welche aus Keramik, insbesondere Oxidkeramik, gefertigt sind angewendet werden. Dazu zählen das Abutment und die prothetische Versorgung, wie Zahnkronen oder Brücken. Bei der prothetischen Versorgung wird das erfindungsgemässe Verfahren insbesondere in jenem Bereich angewendet, welcher zur Verbindung mit dem Abutment oder dem Zahnstumpf vorgesehen ist.

Abutment und Implantat können einteilig oder zweiteilig ausgeführt sein. In der zweiteiligen Variante stellen das Implantat und das Abutment zwei separate Teile dar, die vom Zahnarzt verbunden werden. In der einteiligen Variante ist das Implantat und das Abutment einstückig ausgeführt.

Die Anwendung des erfindungsgemässen Verfahrens ist nicht auf die einteilige oder zweiteilige Variante beschränkt.

Das Ätzmittel, welches erfindungsgemäss zum Einsatz kommt umfasst eine mehrprotonige Säure, mindestens ein Alkalisalz und Wasser.

Bei der mehrprotonigen Säure handelt es sich um eine starke Säure mit einem pKs-Wert ≤4.

Das verwendete Ätzmittel umfasst, in einer bevorzugten Ausführungsform eine Mischung aus Schwefelsäure, Natriumfluorid, Flusssäure und Wasser, wobei sich der Anteil in Gewichtsprozent der einzelnen Ätzmittelkomponenten während der Ätzdauer verändert. Im Zuge des Verfahrens bilden sich HSO₄⁻ Ionen die ebenfalls als Säure wirken und zur Ätzung beitragen.

Das erfindungsgemässe Verfahren basiert auf der Reaktion von Natriumfluorid mit Schwefelsäure, diese lautet allgemein wie folgt:

2NaF + H₂SO₄ → Na₂SO₄ + 2HF

Und genauer:

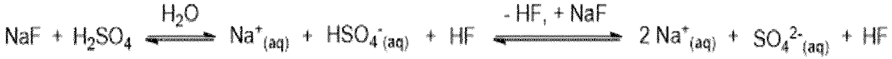

Durch die Verwendung einer Mischung aus Schwefelsäure und Natriumfluorid resultiert als Reaktionsprodukt Flusssäure. Für die Ätzung der Keramik stehen reaktionsgemäss drei Säuren zur Verfügung. Erstens die eingesetzte Schwefelsäure, die während der Reaktion in situ sich bildende Flusssäure und die sich bildenden Hydrogensulfationen.

Beim Ätzverfahren ist der Anteil in Gewichtsprozent der einzelnen Ätzmittelkomponenten abhängig von der Ätzdauer.

Ein Vorteil des erfindungsgemässen Verfahrens liegt darin, dass die Flusssäure nicht direkt angewendet wird, sondern erst als Reaktionsprodukt entsteht, welches sofort reagiert und dabei verbraucht wird. Dies erleichtert insbesondere die Handhabung der Einsatzstoffe im Zahntechniklabor bzw. in der Zahnarztpraxis.

Die Konzentration der verwendeten mehrprotonigen Säure beträgt zwischen 60 Gew. % und 90 Gew. %, vorzugsweise 70 Gew. % oder mehr, besonders bevorzugt 80 Gew. %.

Beispielhaft werden 100 g einer 80%-igen Schwefelsäure aus einer 96%-iger Schwefelsäure hergestellt, in dem bei 20°C, 83.33 g 96%iger Schwefelsäure und 16.67 g H₂O vermischt werden.

Für die Herstellung der gewünschten Schwefelsäurekonzentrationen wird vorzugsweise eine Schwefelsäure mit einer 95-97%-tigen Ausgangskonzentration herangezogen.

Das verwendete Ätzmittel umfasst 2% bis 5% eines flourhaltigen Alkalisalzes, vorzugsweise 3%. In einer bevorzugten Ausführungsform handelt es sich beim Alkalisalz um ein fluorhaltiges Alkalisalz, vorzugsweise Natriumfluorid. Weitere Beispiele für verwendbare fluorhaltige Alkalisalze sind: Kaliumfluorid, Kaliumhydrogendifluorid, Ammoniumfluorid oder Lithiumfluorid.

Die Behandlungstemperatur bei der Durchführung des Verfahrens liegt zwischen 50°C und 100°C, vorzugsweise bei 80°C.

Beim verwendeten Alkalisalz handelt es sich in einer Ausführungsform um NaF mit einer Reinheit von ≥ 99.5%

Die Ätzdauer des Verfahrens, während das Abutment bzw. die prothetische Versorgung dem Ätzmittel ausgesetzt ist, liegt zwischen 12 und 48 Stunden, vorzugsweise beträgt die Dauer 24 Stunden.

Bei der vorliegenden Erfindung ist das Abutment und die prothetische Versorgungaus einem keramischen Werkstoff gefertigt. Bei diesem Werkstoff handelt es sich um eine oxidbasierende Keramik mit Zirkonoxid (ZrO₂), vorzugsweise mit einem Anteil an Yttriumoxid (Y₂O₃) oder mit einem Anteil an Aluminiumoxid (Al₂O₃) und Yttriumoxid (Y₂O₃). Man spricht auch von Hochfesten Keramiken (über 350 MPa Festigkeit), die eine entsprechend hohe Eigenstabilität aufweisen. Der im erfindungsgemässen Verfahren eingesetzte keramische Werkstoff ist gesintert. Das Abutment bzw. eine Innenfläche der prothetischen Versorgung wird vor dem Ätzen mit dem erfindungsgemässen Verfahren nicht weiter behandelt, zum Beispiel weder poliert noch sandgestrahlt.

Eine beispielhafte Zusammensetzung einer solchen Keramik, welche von der Firma Shenyang Upcera Co., Ltd runter dem Produktnamen «Upcera zirconia block» vertrieben wird weist folgende Zusammensetzung auf:
Zirkoniumoxid (ZrO₂) und Hafniumoxid: 94%
Aluminiumoxid < 0.5%
Yttriumoxid < 5.5%

Aufgrund der Behandlung mit dem erfindungsgemässen Ätzverfahren weist jener Teil des Abutments, welcher zur Verbindung mit einer prothetischen Versorgung vorgesehen ist (die sogenannte Haftfläche) eine mesoporöse Oberflächenstruktur auf, vorzugsweise mit einem Porendurchmesser zwischen 2 nm und 50 nm. Vergleichbares gilt für die mit dem erfindungsgemässen Verfahren behandelte Innenfläche der prothetischen Versorgung, beispielsweise der Zahnkrone.

Ferner konnte festgestellt werden, dass jener Teil des Abutments, welcher zur Verbindung mit einer prothetischen Versorgung vorgesehen ist, auch eine makroporöse Oberflächenstruktur zeigt, vorzugsweise mit einem Porendurchmesser grösser 50 nm. Vergleichbares gilt für die mit dem erfindungsgemässen Verfahren behandelte Innenfläche der prothetischen Versorgung, beispielsweise der Zahnkrone.

Es wurde festgestellt, dass bei der Verwendung einer Schwefelsäure mit niedrigerer Konzentration die Keramikoberfläche massiv an geätzt wurde, wohin gegen bei sehr hochprozentiger Schwefelsäure (z.B. 96%iger Konzentration) im Ätzmittel die Aufrauhung der Oberfläche deutlich weniger ausgeprägt war.

### KURZE BESCHREIBUNG DER FIGUREN

Anhand der in den nachfolgenden Figuren gezeigten Ausführungsbeispiele und der dazugehörigen Beschreibung werden Aspekte der Erfindung näher erläutert. Es zeigen:
- Fig. 1: zeigt Fotos von Keramikproben aus Oxidkeramik mit dem Hauptbestandteil ZrO₂ in einem unbehandelten Zustand (a) und behandelten Zustand (b) - (f).
- Fig. 2: zeigt eine Aufnahme aus dem Rasterelektronenmikroskop einer unbehandelten Probe in einer Vergrösserung 1.500-fach (a) und 10.000-fach (b);
- Fig. 3: zeigt eine Aufnahme mit dem Rasterelektronenmikroskop von einer Probe, welche mit dem erfindungsgemässen Ätzverfahren behandelt wurde, in einer Vergrösserung 1.500-fach (a) und 10.000-fach (b);
- Fig. 4: zeigt eine Aufnahme aus dem Rasterelektronenmikroskop von einer Probe welche mit dem erfindungsgemässen Ätzverfahren behandelt wurde, in einer Vergrösserung 1.500-fach (a) und 10.000-fach (b).

### WEGE ZUR AUSFUEHRUNG DER ERFINDUNG

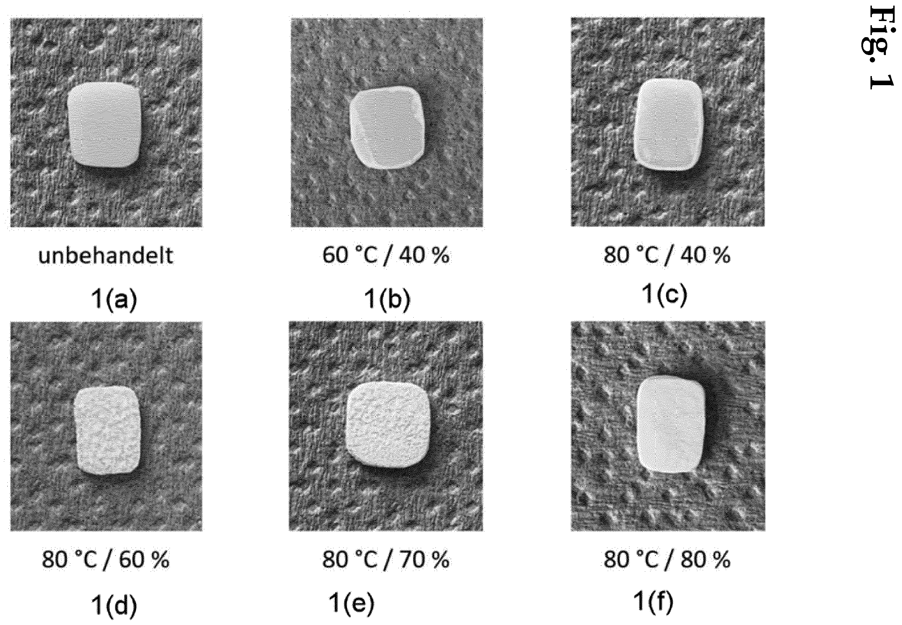

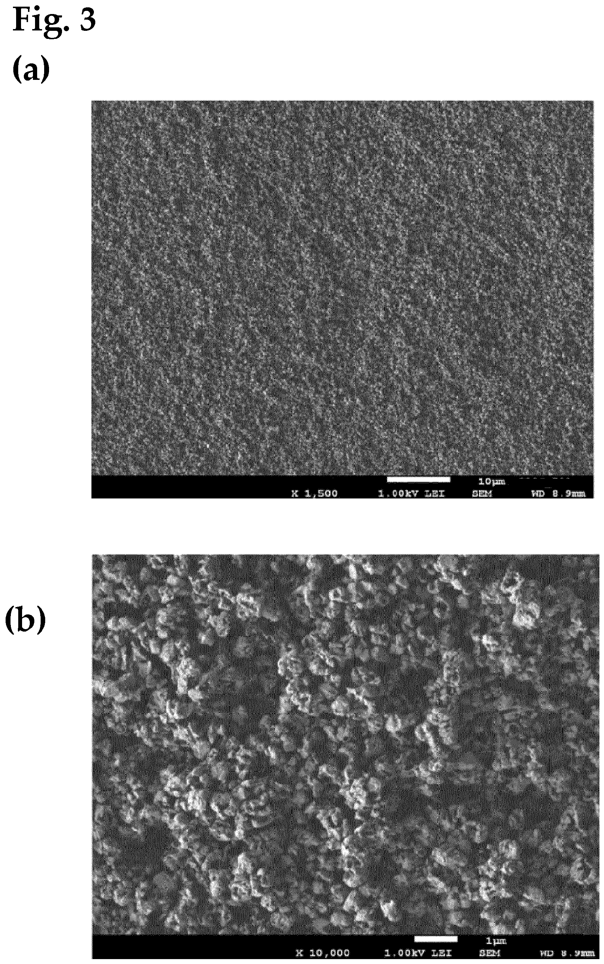

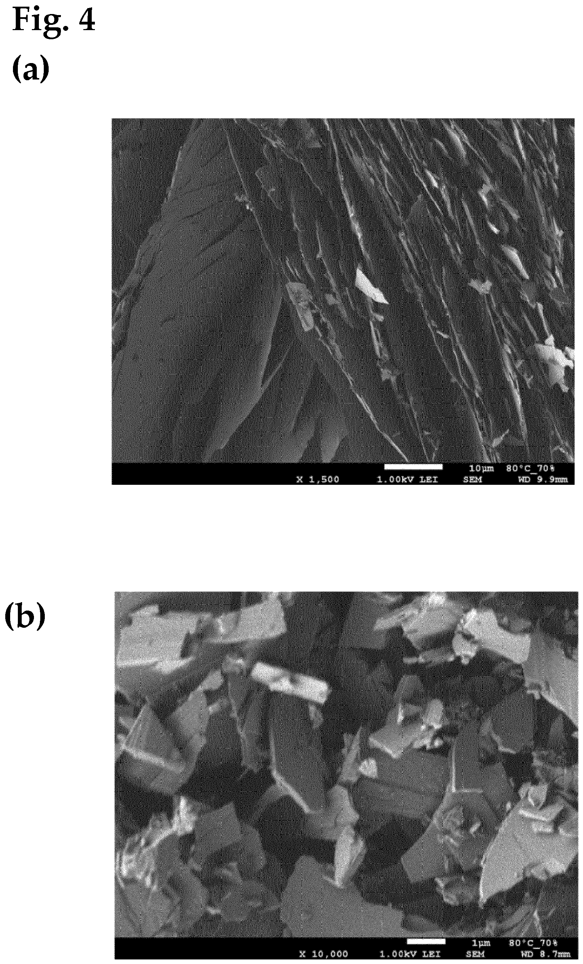

**Figur 1** zeigt Fotos von Keramikproben aus Oxidkeramik mit dem Hauptbestandteil ZrO₂ in einem unbehandelten Zustand (a) und im mit dem erfindungsgemässen Verfahren behandelten Zustand (b) - (f). Bei der Behandlung mit dem erfindungsgemässen Verfahren belief sich die Behandlungsdauer auf 24 Stunden. Bei den Versuchen wurde die Behandlungstemperatur sowie die Edukt-Konzentration der Schwefelsäure (H₂SO₄) variiert. Unter Behandlungstemperatur ist jene Temperatur zu verstehen, die während der Behandlungsdauer (z.B. 24 Stunden) im Wesentlichen konstant eingestellt ist. Das Ätzverfahren wird beispielsweise in einem Ofen ausgeführt. Die Edukt-Konzentration des Alkalisalzes Natriumfluorid (NaF) belief sich bei allen Versuchen (b) - (f) konstant auf 3%. Figur 1 (b) zeigt die Keramikprobe behandelt mit vierzig prozentiger Schwefelsäure und einer Temperatur des Ätzmittels von 60°C. Die Probe wurde unter den gewählten Bedingungen in den Randbereichen an geätzt. Im mittleren Bereich der Probe blieb die Oberfläche unverändert. In Figur 1(c) wurde im Vergleich zu Figur 1(b) die Konzentration der H₂SO₄ mit 40% beibehalten, die Temperatur des Ätzmittels von 60°C auf 80 °C angehoben. Gemäss Fig. 1 (c) wurde ebenfalls der Randbereich an geätzt, der mittlere Bereich der Probe blieb im Wesentlichen unverändert. In den Figuren 1(d)-1(f) wurde die Temperatur des Ätzmittels konstant bei 80°C gehalten und eine sechzig prozentige Schwefelsäure Figur 1(d), eine siebzig prozentige Schwefelsäure (Figur 1 (e)) und eine achtzig prozentige Schwefelsäure (Figur 1 (f)) verwendet. Die Ätzdauer belief sich in allen Versuchen auf 24 Stunden. In allen drei Fällen (d) - (f) ergab sich eine homogene aber unterschiedlich stark ausgeprägte Ätzung der gesamten Oberfläche der Probe. Die Proben aus der Figur 1 (e) und Figur 1(f) wurden unter dem Rasterelektronenmikroskop hinsichtlich ihrer Oberflächenstruktur näher untersucht (Siehe Figur 3 und Figur 4). Es wurde festgestellt, dass bei der Verwendung von siebzig prozentiger Schwefelsäure die Keramikoberfläche massiv angeätzt wurde, wohin gegen bei einer achtzig prozentigen Schwefelsäure im Ätzmittel die Aufrauhung der Oberfläche deutlich weniger ausgeprägt ist.

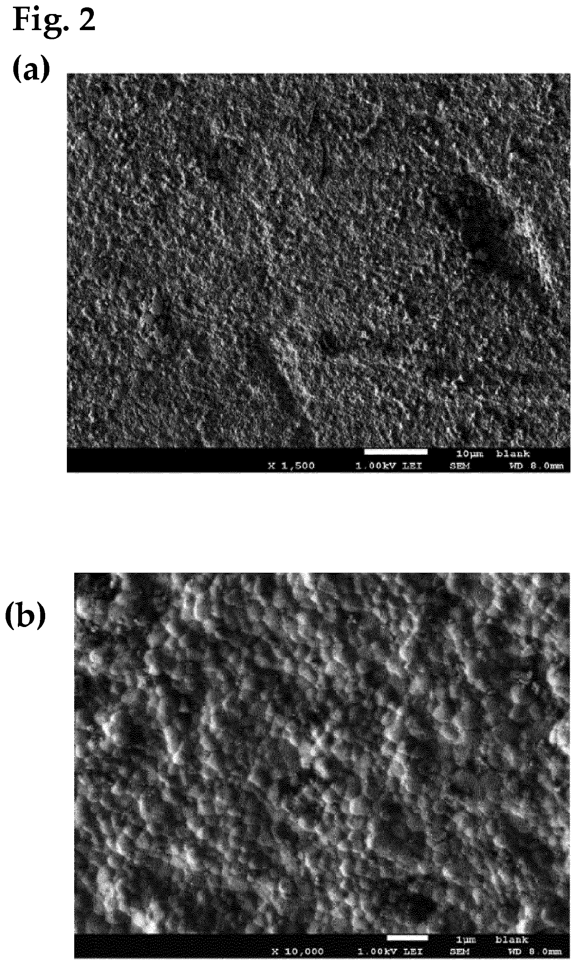

**Figur 2** zeigt eine Aufnahme mit dem Rasterelektronenmikroskop (REM) von einer unbehandelten Probe in einer Vergrösserung 1.500-fach (a) und 10.000-fach (b). Bei der Probe handelt es sich um eine gesinterte Keramik auf Basis von Zirkonoxid (ZrO2). Unbehandelt bedeutet, dass die Probe bevor sie dem erfindungsgemässen Ätzverfahren unterzogen wird, keine vorgängige abrasive Behandlung erfährt, d.h. die dargestellte Probe ist unpoliert und wurde auch nicht mit Hilfe eines Sandstrahlverfahrens vorbearbeitet.
**Figur 3** zeigt eine Aufnahme mit dem Rasterelektronenmikroskop von einer Probe welche mit dem erfindungsgemässen Ätzverfahren behandelt wurde, in einer Vergrösserung von 1.500-fach (a) und 10.000-fach (b). Es handelt sich um eine REM-Aufnahme der Probe aus Figur 1(f). Es wurde eine 80-prozentige Schwefelsäure mit 3% Prozent Natriumfluorid (NaF) eingesetzt. Die Behandlungstemperatur betrug 80°C und die Ätzdauer belief sich auf 24 Stunden. Wie in der Vergrösserung (1 500-fach) gut sichtbar, führt die Behandlung mit dem erfindungsgemässen Verfahren zu einer homogenen Aufrauhung der Proben Oberfläche. Es resultiert eine mesoporöse Struktur.
**Figur 4** zeigt eine Aufnahme mit dem Rasterelektronenmikroskop von einer Probe welche mit dem erfindungsgemässen Ätzverfahren behandelt wurde, in einer Vergrösserung von 1.500-fach (a) und 10.000-fach (b). Es handelt sich dabei um die Probe aus Figur 1(e). Es wurde eine 70% ige-Schwefelsäure mit 3% Natriumfluorid (NaF) eingesetzt. Die Behandlungstemperatur betrug 80°C und die Ätzdauer belief sich auf 24 Stunden. Wie in der Vergrösserung (1500-fach) gut sichtbar, ergibt sich verglichen mit Figur 3 eine andere Oberflächenstruktur nach der Ätzung. Es ist erkennbar, dass es dabei zu einem massiven Angriff der Probenoberfläche unter Ausbildung von plattenförmigen Strukturen kam. Die extreme Rauigkeit ist hier bereits mit dem blossen Auge erkennbar. Der Grad der Verätzung ist stark.

## Patentansprüche

1. Ätzverfahren zur Oberflächenbehandlung einer Zahnersatzkomponente umfassend ein Implantat, ein keramisches Abutment sowie eine keramische prothetische Versorgung, **dadurch gekennzeichnet, dass** eine Innenfläche der prothetischen Versorgung und/oder jener Teil des Abutments, welcher zur Aufnahme der prothetischen Versorgung vorgesehen ist, mit einem Ätzmittel in Kontakt gebracht wird, wobei das Ätzmittel folgende Edukte enthält: eine mehrprotonige Säure, mindestens ein Alkalisalz und Wasser.

2. Ätzverfahren nach Anspruch 1, **dadurch gekennzeichnet, dass** eine Edukt-Konzentration der mehrprotonigen Säure zwischen 60 Gew. % und 90 Gew. % liegt, vorzugsweise mehr als 70 Gew. %, besonders bevorzugt 80 Gew. % beträgt.

3. Ätzverfahren nach einem der vorangegangenen Ansprüche, **dadurch gekennzeichnet, dass** Edukt-Konzentration des Alkalisalzes 2% bis 5%, vorzugsweise 3% beträgt.

4. Ätzverfahren nach einem der vorangegangenen Ansprüche, **dadurch gekennzeichnet, dass** es sich bei mindestens einem Alkalisalz um ein fluorhaltiges Alkalisalz handelt beispielsweise um Natriumfluorid, Kaliumfluorid, Kaliumhydrogendifluorid, Ammoniumfluorid oder Lithiumfluorid, vorzugsweise wird Natriumfluorid eingesetzt.

5. Ätzverfahren nach einem der vorangegangenen Ansprüche, **dadurch gekennzeichnet, dass** es sich bei der mehrprotonigen Säure um eine starke Säure handelt, vorzugsweise mit einem pKs - Wert von kleiner 4.

6. Ätzverfahren nach einem der vorangegangenen Ansprüche, **dadurch gekennzeichnet, dass** es sich bei der mehrprotonigen Säure um Schwefelsäure handelt.

7. Ätzverfahren nach einem der vorangegangenen Ansprüche, **dadurch gekennzeichnet, dass** die Behandlungstemperatur zwischen 50°C und 100°C liegt, vorzugsweise 80°C beträgt.

8. Ätzverfahren nach einem der vorangegangenen Ansprüche, **dadurch gekennzeichnet, dass** die Ätzdauer zwischen 12 Stunden und 48 Stunden beträgt, vorzugsweise 24 Stunden beträgt.

9. Ätzverfahren nach einem der vorangegangenen Ansprüche, **dadurch gekennzeichnet, dass** dem Ätzverfahren keine zusätzlichen mechanischen Behandlungsschritte des keramischen Abutments oder der Innenfläche der prothetischen Versorgung, beispielsweise Sandstrahlen und/oder Polieren vorgelagert sind.

10. Ätzverfahren nach einem der vorangegangenen Ansprüche, **dadurch gekennzeichnet, dass** das im Verfahren eingesetzte keramische Abutment und/oder die prothetische Versorgung einen gesinterten keramischen Werkstoff auf Basis von Zirkonoxid (ZrO₂), vorzugsweise mit einem Anteil an Yttriumoxid (Y₂O₃) oder auf Basis von Zirkonoxid (ZrO₂), vorzugsweise mit einem Anteil an Aluminiumoxid (Al₂O₃) und Yttriumoxid (Y₂O₃) umfasst.

11. Ein Abutment oder eine prothetische Versorgung, **dadurch gekennzeichnet, dass** jener Bereich des Abutments welcher für eine Verbindung, vorzugsweise eine retentive Verbindung mit der prothetischen Versorgung vorgesehen ist und/oder die Innenfläche der prothetischen Versorgung, mit einem Ätzverfahren nach einem der vorangegangenen Ansprüche 1 bis 10 behandelt ist.

12. Das Abutment oder die prothetische Versorgung nach Anspruch 11, **dadurch gekennzeichnet, dass** das Abutment oder die prothetische Versorgung einen gesinterten keramischen Werkstoff auf Basis von Zirkonoxid (ZrO₂), vorzugsweise mit einem Anteil an Yttriumoxid (Y₂O₃) oder auf Basis von Zirkonoxid (ZrO₂), vorzugsweise mit einem Anteil an Aluminiumoxid (Al₂O₃) und Yttriumoxid (Y₂O₃) umfasst.

13. Prothetische Versorgung nach Anspruch 12, **dadurch gekennzeichnet, dass** die prothetische Versorgung an ihrer Innenfläche einen gesinterten keramischen Werkstoff auf Basis von Zirkonoxid (ZrO₂), vorzugsweise mit einem Anteil an Yttriumoxid (Y₂O₃) oder auf Basis von Zirkonoxid (ZrO₂), vorzugsweise in mit einem Anteil an Aluminiumoxid (Al₂O₃) und Yttriumoxid (Y₂O₃) umfasst.

14. Abutment oder die prothetische Versorgung nach einem der Ansprüche 11 bis 13, **dadurch gekennzeichnet, dass** jener Teil des Abutments, welcher zur Verbindung mit einer prothetischen Versorgung vorgesehen ist und/oder die Innenseite der prothetischen Versorgung, nach einer Behandlung mit einen Ätzverfahren gemäss Anspruch 1 bis 10 eine mesoporöse Oberflächenstruktur aufweist, vorzugsweise mit einem Porendurchmesser zwischen 2 nm und 50 nm.

15. Abutment oder die prothetische Versorgung nach einem der Ansprüche 11 bis 14, **dadurch gekennzeichnet, dass** jener Teil des Abutments, welcher zur Verbindung mit einer prothetischen Versorgung vorgesehen ist und/oder die Innenseite der prothetischen Versorgung, nach einer Behandlung mit einen Ätzverfahren gemäss Anspruch 1 bis 10 eine makroporöse Oberflächenstruktur aufweist, vorzugsweise mit einem Porendurchmesser grösser 50 nm.
